Europäisches Patentamt

(19) European Patent Office

Office européen des brevets

(11) Numéro de publication: **0 033 050**
**B1**

(12) FASCICULE DE BREVET EUROPEEN

(45) Date de publication du fascicule du brevet:
16.05.84

(21) Numéro de dépôt: 80401882.8

(22) Date de dépôt: 29.12.80

(51) Int. Cl.³: **G 21 K 1/04,** A 61 N 5/10,
**G 21 G 4/08**

(54) Appareil d'irradiation comprenant un dispositif de collimation secondaire réglable.

(30) Priorité: 11.01.80 FR 8000614

(43) Date de publication de la demande:
05.08.81 Bulletin 81/31

(45) Mention de la délivrance du brevet:
16.05.84 Bulletin 84/20

(84) Etats contractants désignés:
CH DE FR GB IT LI NL

(56) Documents cités:
FR - A - 2 215 777
US - A - 2 542 196
US - A - 2 722 611

(73) Titulaire: C.G.R. MeV, Route de Guyancourt,
F-78530 Buc (FR)
Titulaire: COMMISSARIAT A L'ENERGIE ATOMIQUE
Etablissement de Caractère Scientifique Technique et
Industriel, B.P. 510, F-75752 Paris Cedex 15 (FR)

(72) Inventeur: Perraudin, Claude, THOMSON-CSF
SCPI 173, bld Haussmann, F-75360 Paris Cedex 08 (FR)
Inventeur: Amarge, Edmond, THOMSON-CSF
SCPI 173, bld Haussmann, F-75360 Paris Cedex 08 (FR)
Inventeur: Guiho, Jean-Paul, THOMSON-CSF
SCPI 173, bld Haussmann, F-75360 Paris Cedex 08 (FR)
Inventeur: Horiot, Jean-Claude, THOMSON-CSF
SCPI 173, bld Haussmann, F-75360 Paris Cedex 08 (FR)
Inventeur: Taniel, Gérard, THOMSON-CSF SCPI 173, bld
Haussmann, F-75360 Paris Cedex 08 (FR)
Inventeur: Viel, Georges, THOMSON-CSF SCPI 173, bld
Haussmann, F-75360 Paris Cedex 08 (FR)
Inventeur: Brethon, Jean-Pierre, THOMSON-CSF
SCPI 173, bld Haussmann, F-75360 Paris Cedex 08 (FR)

(74) Mandataire: Barbin le Bourhis, Joel et al,
THOMSON-CSF SCPI 173, boulevard Haussmann,
F-75379 Paris Cedex 08 (FR)

Appareil d'irradiation comprenant un dispositif de collimation secondaire réglable utilisant des sources radioactives

La présente invention se rapporte à un appareil d'irradiation utilisant des sources radioactives telles que le cobalt 60 par exemple et plus particulièrement un dispositif de collimation secondaire destiné à délivrer un faisceau d'irradiation de dimensions précises, facilement réglables, et permettant d'éliminer les zones de pénombre.

Suivant la présente invention, un appareil d'irradiation utilisant au moins une source radioactive et comprenant un support fixe auquel est associé un bras mobile autour d'un axe XX, une tête d'irradiation formée d'une enceinte en matériau imperméable au rayonnement de la source et, dans cette enceinte, un premier blindage de protection biologique entourant au moins partiellement un second blindage de protection biologique, ce second blindage étant muni d'un logement destiné à recevoir la source radioactive, un collimateur primaire destiné à collimater le rayonnement émis par la source radioactive et un système de collimation secondaire du faisceau comportant un pot conique en matériau imperméable au faisceau d'irradiation et, solidaires de ce pot conique, deux paires d'armatures, chacune des armatures, mobile autour d'un axe de rotation fixé sur le pot conique, étant associée à un système mécanique de déplacement, chaque armature étant munie de lames collimatrices, disposées sensiblement perpendiculairement à cette armature, est caractérisé en ce que chaque armature du dispositif de collimation secondaire est reliée au pot conique au moyen d'un système de compas formé de deux étriers s'articulant entre eux autour d'un axe dd, l'un des étrier pouvant tourner autour d'un axe bb fixé sur le pot conique et l'autre étrier pouvant tourner autour d'un axe cc fixé sur l'armature considérée, les axes dd, bb, cc étant parallèles à l'axe de rotation de l'armature sur laquelle est fixé le système de compas.

L'invention sera mieux comprise et d'autres caractéristiques apparaîtront à l'aide de la description ci-après et des dessins qui l'accompagnent et sur lesquels:

— la fig. 1 représente schématiquement un appareil d'irradiation suivant l'invention;
— la fig. 2 représente, en coupe longitudinale, un système de collimation secondaire utilisé dans l'appareil d'irradiation suivant l'invention;
— la fig. 3 montre une vue partielle, en perspective, du système de collimation de la fig. 2;
— la fig. 4 représente un système de déplacement des armatures du collimateur secondaire;
— la fig. 5 représente un système d'élimination de pénombre associé à une armature du collimateur secondaire.

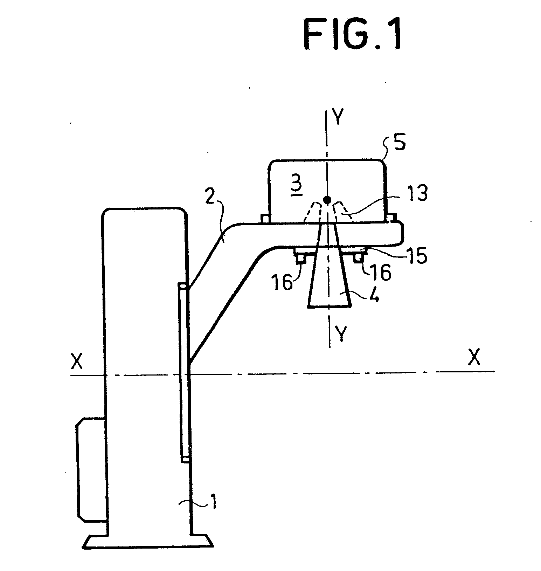

L'appareil d'irradiation montré en fig. 1 et qui peut être utilisé avantageusement en radiothérapie, comporte:

— un support fixe 1;
— un bras 2 mobile autour d'un axe XX;
— une tête d'irradiation 3;
— un collimateur primaire 13;
— un dispositif de collimation secondaire 4 d'axe YY.

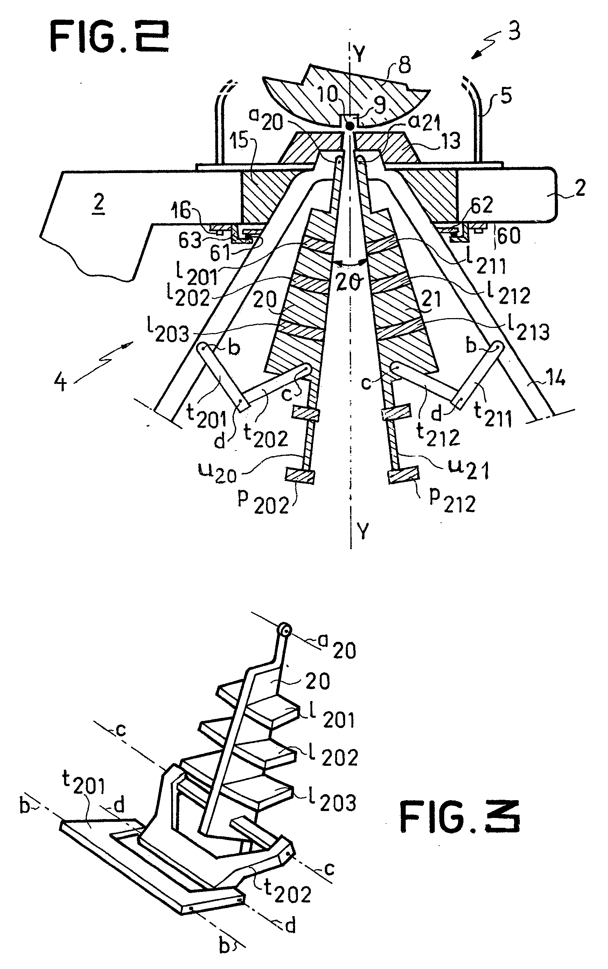

La tête d'irradiation comporte de façon connue, une enceinte 5 dans laquelle sont placés un premier blindage fixe de protection biologique non visible sur la fig. 2 entourant au moins partiellement un second blindage mobile 8 de protection biologique, ce second blindage 8 étant muni d'un logement 9 destiné à recevoir une source radioactive 10. Ce logement 9 s'ouvre vers l'extérieur de blindage 8 de façon à laisser passer le faisceau d'irradiation délivré par la source 10 radioactive.

L'appareil d'irradiation suivant l'invention est muni d'un collimateur 13 primaire d'axe YY, de type connu, et d'un dispositif de collimation secondaire 4 suivant l'invention. Ce dispositif de collimation secondaire 4, fixé sur le bras mobile 2 de façon amovible, au moyen d'un plateau-support 15 par exemple et de vis 16, est décrit dans ce qui suit.

Ce dispositif de collimation secondaire (fig. 2) comprend un pot conique 14 en matériau imperméable aux rayonnements de la source 10 radioactive, deux paires d'armatures 20, 21; 22, 23 (seules les armatures 20, 21 sont visibles sur la fig. 2) solidaires du pot conique 14. Les armatures 20, 21 sont munies respectivement d'une série de lames $1_{201}$, $1_{202}$, $1_{203}$ et $1_{211}$, $1_{212}$, $1_{213}$; ... disposées à la suite les unes des autres et perpendiculaires à l'armature à laquelle elles sont associées.

Les armatures 20, 21, mobiles respectivement autour d'axes $a_{20}$ et $a_{21}$ fixés sur le pot conique 14, sont associées à un système mécanique de déplacement leur permettant de se déplacer simultanément et de façon identique. Il en est de même des armatures 22, 23 (non représentées) disposées dans un plan perpendiculaire au plan formé par les armatures 20, 21. Ces armatures 22, 23 (non visibles sur la fig. 2) sont mobiles autour d'axes identiques à ceux des armatures 20, 21, et un autre système mécanique de déplacement, identique à celui précédemment cité est associé à ces armatures 22, 23.

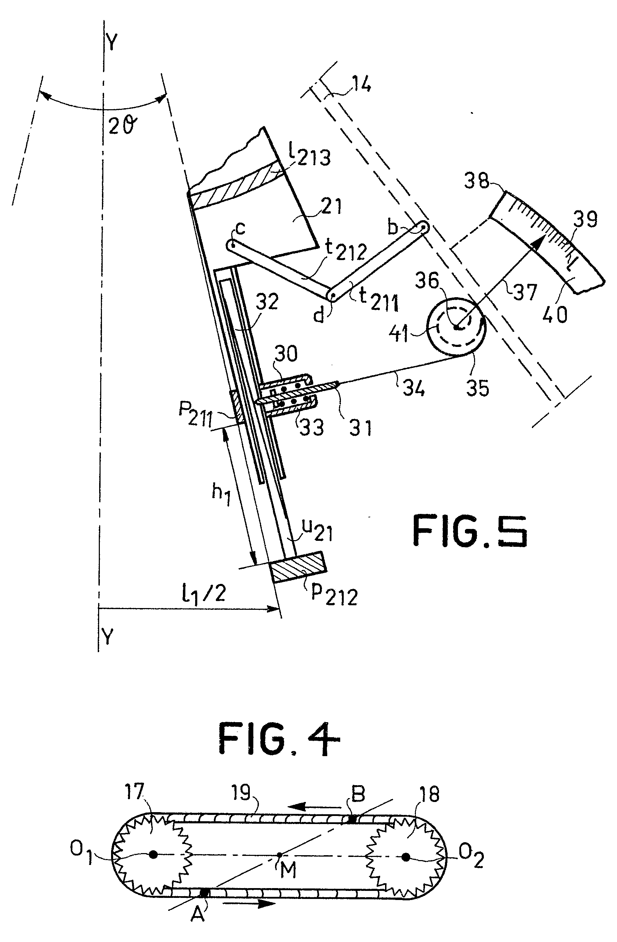

La fig. 4 montre schématiquement, à titre d'exemple de réalisation non limitatif, un système mécanique de déplacement des armatures 20, 21 placées en vis-à-vis. Ce système comprend deux pignons 17, 18 de renvoi, pouvant tourner autour d'axes passant par leur centre $O_1$, $O_2$ respectifs, et une chaîne 19 entourant ces pignons 17, 18. Les armatures 20, 21 sont fixées respectivement sur la chaîne 19 en deux points A et B symétriques par rapport à un point M situé à mi-distance des centres $O_1$, $O_2$. Ces points A et B et donc les armatures 20, 21 se déplacent en sens inverse lorsque le système est mis en fonctionnement.

Afin d'éviter tout jeu préjudiciable au bon fonctionnement du dispositif de collimation chacune de ces armatures 20, 21; 22, 23 du dispositif de collimation secondaire suivant l'invention est associée à un système de compas montré de façon détaillée en fig. 3. Le système de compas associé à l'armature 20 comprend un premier étrier $t_{201}$ mobile autour d'un axe bb fixé sur le pot conique 14, un second étrier $t_{202}$ mobile autour d'un axe cc perpendiculaire à l'armature 20 et fixé sur cette armature 20. Les étriers $t_{201}$ et $t_{202}$ s'articulent entre eux autour d'un axe dd libre. Les seuls mouvements des étriers $t_{201}$, $t_{202}$ sont donc des mouvements de rotation autour des axes bb, cc, dd de telle sorte que les armatures 20, 21 restent coplanaires au cours de leur déplacement.

Des vis pointeaux peuvent assurer le positionnement définitif des bras $t_{201}$, $t_{202}$.

Egalement suivant l'invention, le dispositif de collimation secondaire est muni d'un système d'élimination de pénombre.

En effet, lorsque la source radioactive 10 est de diamètre assez important (1 à 2 cm par exemple) les bords du champ de traitement sont affectés d'une pénombre, conséquence de la forme géométrique du dispositif de collimation secondaire, les deux armatures 20 et 21 en vis-à-vis étant mobiles autour de deux axes $a_{20}$ et $a_{21}$ séparés l'un de l'autre par une distance supérieure au diamètre de la source 10 radioactive.

La fig. 5 montre un détail de ce système d'élimination de pénombre où seule l'armature 21 a été représentée. A cette armature 21 sont associées deux lames $p_{211}$, $p_{212}$ d'élimination de pénombre. La lame $p_{212}$ est fixée à l'extrémité d'une tige $u_{21}$ pouvant coulisser dans l'armature 21 correspondante. La lame $p_{211}$ est fixée sur l'armature 21. L'angle d'ouverture $2\,\theta$ des armatures 20, 21 en vis-à-vis et la position de la lame $p_{212}$ mobile sont vérifiées au moyen d'un système de contrôle de position associé à chaque paires d'armatures 20, 21 (et 22, 23). A chaque angle d'ouverture $2\,\theta$ des armatures associées, correspond une position des lames $p_{202}$ et $p_{212}$ en vis-à-vis (seule la lame $p_{212}$ est visible sur la fig. 5) assurant l'élimination de la pénombre sur les bords du champ de traitement. Comme montré en fig. 5 le système de contrôle de l'angle d'ouverture $2\,\theta$ et de la position de la lame $p_{212}$ comporte un boîtier 30 fixé sur l'armature 21. A l'intérieur de ce boîtier 30 est placé un palpeur 31 venant s'appliquer, au moyen d'un ressort 33, au fond d'une rainure 32 aménagée dans la tige $u_{21}$ et pouvant coulisser dans l'armature 21. Cette rainure 32 est oblique par rapport à l'axe de coulissement. A l'extrémité libre du palpeur 31 est fixée l'une des extrémités d'un fil 34, l'autre extrémité du fil 34 étant fixée à la périphérie d'un tambour 35 de section circulaire pouvant tourner autour d'un axe 36 passant par son centre et fixé sur le pot conique 14. Une aiguille 37 solidaire du tambour 35 peut se déplacer sur un cadran 38 gradué placé à l'extérieur du pot conique 14.

Dans l'exemple de réalisation montré en fig. 5, la lame $p_{211}$ fixe de pénombre est solidaire du boîtier 30.

Un ressort de rappel 41 est placé dans le tambour 35 afin de maintenir tendu le fil 34. Ce ressort 41 est fixé, d'une part, sur l'axe 36 et, d'autre part, sur le tambour 35.

En fonctionnement, à une valeur donnée de l'angle d'ouverture $2\,\theta$ des armatures 20 et 21 et une position donnée des lames $p_{202}$ et $p_{212}$ d'élimination de pénombre (distance des lames $p_{211}$, $p_{212}$ égale à $h_1$) correspond une largeur $l = l_1$ du champ de traitement (la valeur de $l$ est indiquée sur le cadran 38 gradué). Pour le même angle d'ouverture $2\,\theta$ et une autre position des lames $p_{202}$, $p_{212}$, la largeur $l$ du champ de traitement est égale à $l_2$. Le cadran 38 associé à une paire d'armatures 20, 21 par exemple, fournit donc l'une des dimensions (la largeur $l$) du champ de traitement, de forme rectangulaire par exemple. L'autre dimension sera founit par un système de contrôle identique, associé à l'autre paire d'armatures 22, 23 (non représentées).

Dans l'exemple de réalisation décrit, les lames $p_{202}$, $p_{212}$ d'élimination de pénombre placées en vis-à-vis peuvent être positionnées simultanément, de façon identique, soit manuellement, soit par exemple au moyen d'un moteur couplé aux tiges $u_{20}$, $u_{21}$ portant respectivement les lames $p_{202}$, $p_{212}$.

Dans un autre exemple de réalisation, un système de contrôle des dimensions du champ de traitement peut être associé à chacune des armatures 20, 21 (et 22, 23 non représentées), les cadrans gradués 38 associés à ces armatures 20, 21 (et 22, 23) donnant alors respectivement les valeurs des distances $d_1$, $d_2$ séparant deux des bords du champ de traitement à l'axe YY. (Dans ce cas on doit avoir

$$d_1 = d_2 = \frac{l}{2} \Bigg).$$

Enfin, dans l'exemple de réalisation montré en fig. 2, du dispositif de collimation secondaire suivant l'invention, le pot conique 14 peut tourner autour de l'axe YY. En effet, un système de fixation 60 de ce pot conique 14 sur le bras 2 mobile, comporte un roulement 61 inséré entre deux paliers 62, 63, le palier 62 étant solidaire du pot conique 14 et le palier 63 fixé de façon amovible sur le bras 2 mobile.

## Revendications

1. Appareil d'irradiation utilisant au moins une source radioactive (10) et comportant un support (1) fixe auquel est associé un bras (2) mobile autour d'un axe XX, une tête d'irradiation (3) comprenant une enceinte (5) en matériau imperméable au rayonnement de la source et, dans cette enceinte (5), un premier blindage de protection biologique entourant au moins partiellement un second blindage (8) de protection biologique ce second blindage (8) étant muni d'un logement (9) destiné à recevoir la source (10) radioactive, un collimateur primaire (13) et un dispositif de collimation secondaire (4) comportant un pot conique (14) en matériau imperméable au faisceau d'irradiation et, solidaire de ce pot conique (14) d'axe YY, deux paires d'armatures (20, 21; 22, 23), chacune des armatures, mobile autour d'un axe fixé sur le pot conique (14), étant associée à un système mécanique de déplacement, chaque armature (20 . . .) étant munie de lames ($1_{201}$, $1_{202}$, $1_{203}$ . . .) collimatrices disposées sensiblement perpendiculairement à l'armature (20 . . .) sur laquelle elles sont fixées, caractérisé en ce que chaque armature (20 . . .) du dispositif de collimation secondaire est reliée au pot conique (14) au moyen d'un système de compas formé de deux étriers ($t_{201}$, $t_{202}$) s'articulant entre eux autour d'un axe dd, l'un des étriers ($t_{201}$) pouvant tourner autour d'un axe bb fixé sur le pot (14) conique et l'autre étrier ($t_{202}$) pouvant tourner autour d'un axe cc fixé sur l'armature (20 . . .) considérée, les axes (dd, bb, cc) étant parallèles à l'axe ($a_{20}$) de rotation de l'armature (20) sur laquelle est fixé le système de compas.

2. Appareil d'irradiation suivant la revendication 1, caractérisé en ce que le système mécanique de déplacement de deux armatures (20, 21) placées en vis-à-vis comporte deux pignons (17, 18) de renvoi pouvant tourner autour d'axes passant par leur centre ($O_1$, $O_2$) et une chaîne (19) entourant les pignons (17, 18) et en ce que les armatures (20, 21) sont fixées respectivement sur la chaîne (19) en deux points (A, B) symétriques par rapport à un point (M) situé à midistance des centres ($O_1$, $O_2$).

3. Appareil d'irradiation suivant la revendication 1, caractérisé en ce que le dispositif de collimation secondaire est muni d'un système d'élimination de pénombre comportant, associée à chaque armature (21 . . .), une lame $p_{212}$ fixée sur une tige ($u_{21}$) qui peut coulisser respectivement dans l'armature (21) correspondante et en ce que des systèmes de contrôle permettant de déterminer l'angle d'ouverture 2 $\theta$ des armatures (20, 21; . . .) en vis-à-vis ainsi que la position des lames d'élimination de pénombre sont associés aux paires d'armatures (20, 21; . . .).

4. Appareil d'irradiation suivant la revendication 3, caractérisé en ce que le système de contrôle de la position des armatures (20, 21 . . .) et des lames d'élimination de pénombre $p_{201}$, $p_{212}$ correspondantes comporte, associé au moins à une paire d'armatures (20, 21; . . .) en vis-à-vis: un boîtier (30) fixé sur l'armature (21), dans ce boîtier (30) étant placé un palpeur (31) venant s'introduire dans une rainure (32), oblique par rapport à l'axe de coulissement, d'une tige ($u_{21}$) coulissant dans l'armature (21), un ressort (33) destiné à appliquer ce palpeur (31) au fond de la rainure (32) oblique, en ce que, à l'extrémité libre du palpeur (31) est fixée l'une des extrémités d'un fil (34), l'autre extrémité de ce fil (34) étant fixée à la périphérie d'un tambour (35) circulaire pouvant tourner autour d'un axe (36) passant par son centre, et en ce qu'une aiguille (37) est fixée sur le tambour, perpendiculairement à l'axe (36), cette aiguille (37) pouvant se déplacer sur un cadran 38 gradué.

5. Appareil d'irradiation suivant l'une quelconque des revendications 3 et 4, caractérisé en ce que le pot conique (14) est solidaire d'un plateau-support (15) destiné à être fixé au moyen de vis (16) sur le bras mobile (2).

6. Appareil d'irradiation suivant la revendication 5, caractérisé en ce que le pot conique (14) est associé à un système de fixation (60) permettant sa fixation amovible sur le bras mobile (2), ce système de fixation (60) comprenant un plateau disposé perpendiculairement à l'axe YY et formant un palier (62) solidaire du pot conique (14), un palier (63) fixé de façon amovible sur le bras mobile (2) et, entre les paliers (62, 63), un roulement (61).

## Patentansprüche

1. Bestrahlungsgerät mit wenigstens einer Radioaktiv-Strahlungsquelle (10) und einem ortsfesten Träger (1), dem ein um eine XX-Achse beweglicher Arm (2) beigeordnet ist, ferner ein eine aus einem für die Strahlung der Quelle undurchlässigen Material bestehenden Hülle (5) umfassenden Bestrahlungskopf (3), und ein in dieser Hülle (5) angeordneter, einen zweiten biologischen Schutzpanzer (8) wenigstens teilweise umgebenden ersten biologischen Schutzpanzer, wobei dieser zweite Panzer (8) einen Sitz (9) zur Aufnahme der Radioaktiv-Strahlungsquelle (10) aufweist, wobei ein Primärkollimator (13) und eine Sekundärkollimationsvorrichtung (4) mit einem kegelförmigen Topf (14) aus für das Strahlenbündel undurchlässigem Material vorgesehen sind und mit dem eine YY-Achse besitzenden kegelförmigen Topf (14) zwei Garniturenpaare (20, 21; 22, 23) fest verbunden sind und jede dieser um eine an dem kegelförmigen Topf (14) befestigte Achse beweglichen Garnituren mit einem mechanischen Verstellungssystem zusammenwirkt und ferner jede Garnitur (20 . . .) mit an der Garnitur (20 . . .) befestigten und sich senkrecht zu derselben erstreckenden Kollimationsblättern ($i_{201}$, $i_{202}$, $i_{203}$ . . .) versehen ist, dadurch gekennzeichnet, daß jede Garnitur (20 . . .) der Sekundärkollimationsvorrichtung mit dem kegelförmigen Topf (14) vermittels eines aus zwei durch eine Achse dd aneinander angelenkten Schenkeln ($t_{201}$, $t_{202}$) bestehenden Gabelsystems mit dem kegelförmigen Topf (14) verbunden ist, wobei einer der Schenkel ($t_{201}$) um eine am kegelförmigen Topf (14) befestigte Achse bb und der andere Schenkel ($t_{202}$) um eine an der betreffenden Garnitur (20 . . .) befestigte Achse cc drehbar ist, während diese Achsen (dd, bb, cc) zur Drehachse ($a_{20}$) der Garnitur (20) parallel sind, an welcher das Gabelsystem befestigt ist.

2. Bestrahlungsgerät nach Anspruch 1, dadurch gekennzeichnet, daß das mechanische Verstellungssystem für zwei einander gegenüberliegende Garnituren (20, 21) zwei Umlenkzahnräder (17, 18) umfaßt, die um durch deren Mittelpunkte ($O_1$, $O_2$) verlaufende Achsen drehbar sind, sowie eine um die Zahnräder (17, 18) laufende Kette (19), und daß die Garnituren (20, 21) an der Kette (19) an je einem von zwei Punkten (A, B) befestigt sind, die in bezug auf einen in der Mitte zwischen den Mittelpunkten ($O_1$, $O_2$) gelegenen Punkt (M) symmetrisch angeordnet sind.

3. Bestrahlungsgerät nach Anspruch 1, dadurch gekennzeichnet, daß die Sekundärkollimationsvorrichtung ein Halbschatteneliminationssystem aufweist, das ein je einer Garnitur (21 . . .) zugeordnetes Blatt $p_{212}$ umfaßt, welches an einer jeweils in der betreffenden Garnitur (21) gleitbar angeordneten Stange ($u_{21}$) befestigt ist, und daß zur Bestimmung des Öffnungswinkels $2\,\Theta$ der einander gegenüberliegend angeordneten Garnituren (20, 21; . . .), sowie zur Bestimmung der Lage der Halbschatteneliminationsblätter den Garniturpaaren (20, 21; . . .) Kontrollsysteme beigeordnet sind.

4. Bestrahlungsgerät nach Anspruch 3, dadurch gekennzeichnet, daß das Lagenkontrollsystem für die Garnituren (20, 21 . . .) und für die entsprechenden Halbschatteneliminationsblätter $p_{201}$, $p_{212}$ in Verbindung mit wenigstens einem Paar sich gegenüberliegender Garnituren (20, 21; . . .) folgende Bauteile umfaßt: ein an der Garnitur (21) befestigtes Gehäuse (30), in welchem ein Fühler (31) angeordnet ist, der in eine in bezug auf die Gleitachse schräg angeordnete Nut (32) einer in der Garnitur (21) gleitenden Stange ($u_{21}$) eingreift, sowie eine den Fühler (31) an den Boden der schrägen Nut (32) andrückende Feder (33), daß am freien Ende des Fühlers (31) ein Ende eines Drahtes (34) befestigt ist, dessen anderes Ende am Umfang einer kreisförmigen Trommel (35) befestigt ist, die um eine durch deren Mittelpunkt verlaufende Achse (36) drehbar ist, und daß senkrecht zur Achse (36) an der Trommel eine auf einer graduierten Skala (38) bewegliche Nadel (37) befestigt ist.

5. Bestrahlungsgerät nach einem der Ansprüche 3 und 4, dadurch gekennzeichnet, daß der kegelförmige Topf (14) mit einer Tragplatte (15) fest verbunden ist, die vermittels Schrauben (16) an dem beweglichen Arm (2) befestigbar ist.

6. Bestrahlungsgerät nach Anspruch 5, dadurch gekennzeichnet, daß der kegelförmige Topf (14) einem Befestigungssystem (60) beigeordnet ist, vermittels dessen er lösbar am beweglichen Arm (2) anbringbar ist und das eine senkrecht zur Achse YY angeordnete Platte umfaßt, welche ein mit dem kegelförmigen Topf (14) fest verbundenes Lagerelement (62) bildet, sowie ein lösbar am beweglichen Arm (2) angebrachtes Lagerelement (63) und ferner ein zwischen den Lagerelementen (62, 63) angeordnetes Wälzlager (61).

**Claims**

1. An irradiating apparatus using at least one radioactive source (10) and comprising a fixed support (1) associated to an arm (2) movable about an axis XX, an irradiating head (3) including an enclosure (5) made of a material impervious to the radiation of the source and, within said enclosure (5), a first shielding for biological protection which at least partially surrounds a second shielding (8) for biological protection, said second shielding (8) being provided with a recess (9) adapted to receive the radioactive source (10), a primary collimator (13) and a secondary collimating device (4) comprising a conical pot (14) made of a material impervious to the irradiation beam, and, integral with said conical pot (14) which has an axis YY, two pairs of armatures (20, 21; 22, 23) each one of which is movable about an axis fixed to the conical pot (14) and each one of which is associated to a mechanical displacing system, each armature (20 . . .) being provided with collimating blades ($1_{201}$, $1_{202}$, $1_{203}$ . . . ) disposed substantially perpendicularly to the armature (20 . . .) on which they are fixed, characterized in that each armature (20 . . .) of the secondary collimating device is connected to the conical pot (14) by means of a fork system constituted by two braces ($t_{201}$, $t_{202}$) hingedly connected to each other by an axis dd, one of said braces ($t_{201}$) being able to rotate about an axis bb fixed to the conical pot (14), while the other brace ($t_{202}$) is able to rotate about an axis cc fixed to the corresponding armature (20 . . .), said axes (dd, bb, cc) being parallel to the axis ($a_{20}$) of rotation of the armature (20) to which said fork system is fixed.

2. An irradiating apparatus according to claim 1, characterized in that the mechanical system for displacing two mutually opposed armatures (20, 21) comprises two reversing pinions (17, 18) rotatable about axes which extend through their centre ($O_1$, $O_2$) and a chain (19) surrounding said pinions (17, 18) and in that the armatures (20, 21) are fixed respectively to the chain (19) at two points (A, B) symmetrically disposed with respect to a point (M) located at half-distance between said centres ($O_1$, $O_2$).

3. An irradiating apparatus according to claim 1, characterized in that said secondary collimating device is provided with a penumbra eliminating system comprising, in association with each armature (20, . . .) a blade $p_{121}$ fixed onto a rod ($u_{21}$) able to slide respectively in the corresponding armature (21), and in that control systems for determining the corner angle $2\,\Theta$ of the mutually opposed armatures (20, 21; . . .) and the position of the penumbra eliminating blades are associated to the pairs of armatures (20, 21; . . .).

4. An irradiating apparatus according to claim 3, characterized in that the system for controlling the position of the armatures (20, 21; . . .) and that of the corresponding penumbra eliminating blades $p_{201}$, $p_{212}$ comprises, in association with at least one pair of opposed armatures (20, 21; . . .): a casing (30) fixed to the armature (21), said casing enclosing a feeler (31) penetrating a groove (32) which is provided obliquely with respect to the sliding axis in a rod ($u_{21}$) slideably mounted in the armature (21), a spring (33) adapted to apply said feeler into the bottom of the oblique groove (32), in that one of the ends of a wire (34) is attached to the free end of the feeler (31), while the other end of said wire (34) is attached to the periphery of a circular drum (35) adapted to rotate about an axis (36) extending through its centre, and in that a needle (37) is affixed to the drum perpendicularly to the axis (36), said needle (37) being displaceable on a graduated dial (38).

5. An irradiating apparatus according to claim 5, characterized in that said conical pot (14) is integral with a supporting plate (15) adapted to be fixed to the movable arm (2) by means of screws (16).

6. An irradiating apparatus according to claim 5, characterized in that said conical pot (14) is associated to a fixing system (60) enabling it to be removably fixed to the movable arm (2), said fixing system (60) comprising a plate disposed perpendicularly to the axis YY and constituting a bearing member (62) integral with the conical pot (14), a bearing member (63) removably fixed to the movable arm (2), as well as a rolling-contact bearing (61) provided between the bearing members (62, 63).

# FIG.1

# FIG.2

# FIG.3

FIG.5

FIG. 4